# EUROPÄISCHE PATENTSCHRIFT

(11) **EP 2 338 425 B1**
(45) Veröffentlichungstag und Bekanntmachung des Hinweises auf die Patenterteilung: **24.10.2012**
(21) Anmeldenummer: 10015598.5
(22) Anmeldetag: 14.12.2010
(51) Int. Cl.: A61B 17/34, A61B 19/00

(54) **Trokarhülse**
Trocar sleeve
Enveloppe de Torkar

(30) Priorität: 24.12.2009 DE 102009060377
(43) Veröffentlichungstag der Anmeldung: 29.06.2011
(73) Patentinhaber: Richard Wolf GmbH, 75438 Knittlingen (DE)
(72) Erfinder: Boebel, Manfred, 75245 Bauschlott (DE); Bonnet, Ludwig, 75438 Knittlingen (DE); Körner, Eberhard, Dipl.-Ing., 75438 Knittlingen (DE); Raakow, Roland, Dr., 14163 Berlin (DE); Liesaus, Georg, Dr., 14469 Potsdam (DE)
(74) Vertreter: Vollmann, Heiko

(56) Entgegenhaltungen:
- WO-A1-00/18306
- DE-A1-102005 026 467
- US-A1- 2003 055 437
- US-A1- 2008 058 723
- US-A1- 2008 255 519

## Beschreibung

### Beschreibung

Die Erfindung betrifft eine Trokarhülse mit den im Oberbegriff des Anspruchs 1 angegebenen Merkmalen sowie im Weiteren den zugehörigen Trokar.

Der Trokar ist ein Instrument, mit dessen Hilfe in der minimalinvasiven Chirurgie ein Zugang zu einer Körperhöhle, insbesondere dem Bauchraum, geschaffen wird. Zum Trokar gehört eine Trokarhülse, die zusammen mit dem Trokar eingesetzt wird und die den späteren Zugang in die Körperhöhle offenhält. Der Trokar wird zusammen mit der Trokarhülse, beispielsweise durch die Bauchdecke eingeführt, wonach der Trokar herausgezogen und über die verbleibende Trokarhülse das Einführen von Instrumenten, beispielsweise Optiken-, Greif-, Schneid- oder anderen Werkzeugen erfolgen kann.

Um die Trokarhülse z. B. in der Bauchdecke zu befestigen, zählt es zum Stand der Technik, diese mit einem geeigneten Gewinde, d. h. mit einer schraubenlinienförmig angeordneten Wendel am distalen Ende zu versehen. Derartige Trokarhülsen gemäß dem Oberbegriff des Anspruchs 1, sind aus US 2008/0255519 A1 bekannt. In dieser Druckschrift ist insbesondere anhand der Fig. 24 und 25 eine Trokarhülse beschrieben, deren distaler Endabschnitt an der Außenseite mit einer Wendel versehen ist und der proximalseitig in einen trichterförmig aufweitenden Bereich übergeht. Durch das Gewinde soll die Trokarhülse zuverlässig in der Wandung der Körperhöhle, z. B. der Bauchdecke unter Komprimierung derselben festgesetzt werden. Die Instrumente sind dabei durch den vergleichsweise engen zylindrischen Innenquerschnitt einzuführen, dort ist für ein Laparoskop oder eine Endoskopoptik eine Führung im distalen Endabschnitt vorgesehen, die jedoch den verbleibenden Querschnitt und damit das Einführen weiterer Instrumente zusätzlich erschwert. Auch ist diese Trokarhülse zur Aufnahme eines Trokars nicht geeignet.

Aus DE 10 2005 026 467 A1 ist eine Trokarhülse in Form eines konischen Einsatzes bekannt, die eine Wendel am Außenumfang aufweist und an der Innenseite eine Führung für ein Instrument. Da die Führung parallel zur konischen Wandung angeordnet ist, kann das Instrument nur in dieser Richtung eingeführt und gehalten werden. Durch die Schrägführung bleibt der am distalen Ende zum Durchtritt in die Körperhöhle gebildete kreisrunde Freiraum zwar weitgehend frei von der Führung, doch bedingt die wandungsprallele Anordnung eine Schrägführung der Instrumente, wodurch der Zugang praktisch ebenfalls verengt wird. Die konische Außenseite mit der darauf angeordneten Wendel bietet darüber hinaus keinen genügenden Halt, da eine Komprimierung der Bauchdecke bei dieser Anordnung nicht erfolgen kann und somit ein sicherer Halt nicht gewährleistet ist.

Vor diesem Hintergrund liegt der Erfindung die Aufgabe zugrunde, eine Trokarhülse zu schaffen, welche die vorbeschriebenen Nachteile nicht aufweist und bei vergleichsweise kleinem Querschnitt einen verbesserten Zugang für Instrumente sowie einen verbesserten Halt in der eingeführten Körperöffnung gewährleistet.

Diese Aufgabe wird gemäß der Erfindung durch eine Trokarhülse mit den in Anspruch 1 angegebenen Merkmalen gelöst. Vorteilhafte Ausgestaltung der Erfindung sind in den Unteransprüchen, der nachfolgenden Beschreibung und der Zeichnung dargestellt.

Die erfindungsgemäße Trokarhülse weist einen distalen rohrförmigen Abschnitt und einen proximalseitig daran anschließenden und proximalwärts aufgeweiteten Abschnitt auf. Dabei ist am Umfang des distalen Rohrabschnitts mindestens eine schraubenlinienförmig angeordnete Wendel vorgesehen, die in Richtung der Längsachse der Trokarhülse gesehen eine kreisrunde Außenkontur aufweist. Gemäß der Erfindung weist der Rohrabschnitt einen gerundeten polygonen Querschnitt auf.

Grundgedanke der vorliegenden Erfindung ist es somit, den rohrförmigen Abschnitt, der in den Körper eindringt und typischerweise in der Bauchdecke verankert wird, nicht, wie sonst üblich, mit einem kreisrunden, sondern mit einem gerundeten polygonförmigen Querschnitt auszustatten. Unter gerundetem polygonförmigem Querschnitt ist ein solcher zu verstehen, der an sich polygonförmig ist, bei dem jedoch benachbarte Seiten des Profils nicht als Ecke, sondern als Rundung ausgebildet sind. Der distale rohrförmige Abschnitt ist in an sich bekannter Weise durch ein vergleichsweise dünnwandiges Rohr gebildet, wobei der Rohrquerschnitt nicht wie üblich kreisrund oder oval, sondern polygon ausgebildet ist. Dabei ist jedoch nur dieser distale rohrförmige Abschnitt in dieser Form ausgebildet, die umgebende schraubenlinienförmig angeordnete Wendel hingegen weist eine kreisrunde Außenkontur auf.

Diese erfindungsgemäße Ausbildung ist besonders vorteilhaft, da bei gleicher Querschnittsfläche ein polygoner Querschnitt hinsichtlich der Anordnung und Bewegungsfreiheit der durchzuführenden Instrumente günstiger ist als ein kreisrunder Querschnitt. Die Schwenkbarkeit der Instrumente, also deren Schrägstellbarkeit bezogen auf die Trokarhülsenlängsachse wird deutlich verbessert, darüber hinaus behindern sich die Instrumente gegenseitig nur in geringerem Maße. Ein weiterer wesentlicher Vorteil ist die im Vergleich zum Stand der Technik größere Tragfläche der Wendel, über welche das Körpergewebe, also insbesondere die Bauchdecke aufgenommen und somit die Trokarhülse festgelegt wird. Dadurch, dass aufgrund der runden Außenkontur und des polygonen Rohrquerschnitts sich unterschiedliche wirksame Breiten der Wendel ergeben, können die parallel zu den Seiten des Polygons liegenden Wendelabschnitte breiter ausgebildet sein als bei einem kreisrunden Innenrohr mit vergleichbaren Abmessungen, wodurch eine bessere Komprimierung der Bauchdecke und ein sicherer Halt der Trokarhülse innerhalb der Bauchdecke erzielt werden kann.

Zweckmäßigerweise ist oder sind die Wendel über ihre gesamte Länge abstandsfrei an dem Außenumfang des distalen Rohrabschnittes angeschlossen, so dass die Wendel eine Art Außengewinde mit sich ändernder Flankenbreite bildet.

Als besonders vorteilhaft hat es sich erwiesen, den distalen Rohrabschnitt mit einem gerundeten dreieckigen Querschnitt zu versehen, also derart, dass flache, typischer- aber nicht notwendigerweise im Winkel von 120° zueinander angeordnete Rohrwandungen gebildet sind, die durch entsprechend gerundete Abschnitte zu einem Rohr verbunden sind, derart, dass die Wanddicke des Rohres sowohl über den Umfang als auch über die Länge konstant ist und sich ein stetiger Querschnittsverlauf ergibt, so wie dies typischerweise zur Schaffung eines gasdichten Zugangs z. B. über den Nabel zweckmäßig ist. Dabei ergibt der gerundete dreieckige Querschnitt des rohrförmigen Abschnitts einerseits maximale Freiheitsgrade für die hindurch geführten Hilfsinstrumente andererseits auch maximale Tragflächen des oder der Wendel, nämlich dort, wo diese an die Seiten des Dreiecksquerschnitts anschließen.

Um nach Schaffung des Zugangs und Entfernen des Trokars das bei solchen Eingriffen typischerweise aufgebaute Pneumoperitoneum aufrechterhalten zu können, sind gemäß einer Weiterbildung der Erfindung proximalseitig Mittel zum lösbaren oder verschwenkbaren Anbringen von Dichtmitteln, insbesondere einer Dichtkappe vorgesehen, welche die Trokarhülse proximalseitig nach außen hin abdichtet und mindestens eine selbsttätig schließende oder verschließbare Öffnung zum Durchführen eines Instrumentes aufweist. Diese Dichtung ist vorteilhaft lösbar oder verschwenkbar angebracht, damit sie beim Einführen bzw. Entfernen des Trokars nicht hinderlich ist, zum anderen damit sie gegebenenfalls an den jeweiligen Instrumentensatz angepasst und austauschbar ist sowie aus hygienischen Gründen, damit sie gegebenenfalls als Einwegartikel steril verpackt bereitgestellt werden kann.

Vorteilhaft kann dies dadurch realisiert werden, dass gemäß einer Weiterbildung der Erfindung an dem aufgeweiteten Abschnitt der Trokarhülse proximalseitig ein Endabschnitt vorgesehen ist, welcher mit einem umlaufenden Wulst versehen ist, der von der Dichtkappe übergriffen ist. Das Übergreifen des Wulstes durch die Dichtkappe hat anwendungstechnisch mehrere Vorteile. Zum einen ist eine solche Dichtung für den Anwender schnell und einfach anbringbar bzw. entfernbar. Darüber hinaus wird der proximale Rand der Trokarhülse durch die Dichtkappe übergriffen, wodurch Verletzungen im Bereich dieser Kante, wie sie bei trichterförmigen Trokaren dieser Art gelegentlich auftreten, vermieden oder zumindest verringert werden können. Der umlaufende Wulst, welcher die Dichtkappe auf der Trokarhülse hält, kann durch Einstechen einer Nut gebildet sein oder auch durch Anformen an den proximalseitigen Endabschnitt. Die Nutanordnung hat darüber hinaus den Vorteil, dass die Dichtwirkung nicht nur durch Übergreifen des Wulstes, sondern durch Anlage des übergreifenden Kappenrandes in der Nut erzielt wird und darüber hinaus die Dichtkappe besser gehalten wird.

Darüber hinaus kann der proximale Endabschnitt vorteilhaft zur Anbringung eines beispielsweise stabförmigen Halters vorgesehen sein, der radial an der Außenseite des Endabschnitts anschließt, mit geringem Abstand hierzu um etwa 90° abgebogen ist und dann parallel zur Längsachse der Trokarhülse proximalwärts geführt ist. Auf diesem Halter ist vorteilhaft verstellbar ein Halterarm angeordnet, der zur Führung und Fixierung eines durch die Trokarhülse geführten Instrumentes ausgebildet ist. Zweckmäßigerweise dient dieser Haltearm zur Führung und Festlegung einer Endoskopoptik, kann jedoch auch grundsätzlich zur Festlegung anderer Instrumente genutzt werden. Der Haltearm kann darüber hinaus auch zur Fixierung des Trokars in der Trokarhülse dienen, was besonders vorteilhaft ist.

Wenn, was gemäß einer Weiterbildung der Erfindung vorgesehen ist, der Haltestab zumindest abschnittsweise hohl ausgebildet ist, dann kann über diesen eine Leitungsverbindung in das Innere der Trokarhülse gebildet werden, wenn ein entsprechender und vorzugsweise absperrbarer Leitungsanschluss am Haltestab vorgesehen oder zumindest von diesem abgezweigt ist. Über einen solchen Leitungsanschluss kann das Pneumoperitoneum unterstützt oder aufgebaut werden.

Besonders vorteilhaft ist es, wenn die erfindungsgemäße und vorbeschriebene Trokarhülse mit einem Trokar versehen ist, der, wie an sich bekannt, innerhalb der Trokarhülse festlegbar ist und der einen distal zur einer Spitze hin verjüngten Endabschnitt aufweist. Dabei ist gemäß einer Weiterbildung der Erfindung die Außenkontur des Trokars, insbesondere in dem weitesten Bereich des verjüngten Endabschnitts an die Außenkontur des Innenquerschnitts des distalen Rohrabschnitts der Trokarhülse angepasst, der in diesem Bereich an seiner Außenseite mit mindestens einer Wendel versehen ist. Dabei weist auch dieser zu einer Spitze verjüngte Endabschnitt eine entsprechende Anzahl von Wendeln wie der distale Rohrabschnitt auf, die ebenfalls schraubenlinienförmig von der Spitze nach proximalwärts ansteigend angeordnet sind und die bündig oder proximalwärts versetzte Wendel am Umfang des distalen Rohrabschnitts anschließen, wenn der Trokar in der Trokarhülse in seiner bestimmungsgemäßen Stellung zur Schaffung eines Zugangs durch die Bauchdecke festgelegt ist. Dabei ist es analog zu der Wendelanordnung und Querschnittsform im Bereich des distalen Endabschnitts besonders zweckmäßig, wenn der Trokar im Bereich seines zur Spitze hin verjüngten Endabschnitts einen kreisrunden Querschnitt aufweist, also eine Kegelform aufweist, wohingegen der oder die Wendel in ihrer Breite entsprechend der Innenkontur des umgebenden distalen Rohrabschnitts der Trokarhülse ausgebildet ist. Dann ergibt sich im Bereich des zu einer Spitze hin verjüngten Endabschnitts des Trokars einerseits ein leichtes Eindringen in den Körper, wobei aufgrund der zumindest abschnittsweise vergleichsweise breiten Wendeln eine hohe Tragfähigkeit in diesem Bereich gegeben ist, die auch schon nach geringer Eindringtiefen ein Abrutschen des Trokars sicher verhindert. Darüber hinaus ist der Trokar mit seinem zu einer Spitze hin verjüngten Endabschnitt in seiner bestimmungsgemäßen Einführstellung vorteilhaft so angeordnet, dass er die Trokarhülse, insbesondere im Bereich des distalen Rohrabschnitts nicht vollständig durchdringt, so dass ein Formschluss zwischen dem proximalen Teil dieses Endabschnitts und dem distalen Teil des distalen Rohrabschnitts der Trokarhülse gebildet ist, so dass ein Drehmoment von der Trokarhülse auf den Trokar übertragen werden kann, was zum Einführen besonders vorteilhaft ist.

Alternativ zum bündigen Anschließen von der Wendel der Trokars an die Wendel der Trokarhülse ist das distale Wendelende der Trokarhülse nach distalwärts leicht versetzt angeordnet, um zu verhindern, dass beim Eindrehen der Trokarhülse in die Bauchdecke Gewebe in den Übergangsbereich der Wendel zwischen Trokar und Trokarhülse gelangen kann.

Der Trokar selbst ist proximalseitig an dem verjüngten Endabschnitt mit einem daran anschließenden Stab versehen, der endseitig, also am proximalen Ende eine Handhabe aufweist. Dabei ist der Stab so dimensioniert, dass die Handhabe die Trokarhülse proximalseitig überragt, wenn der Trokar in der Trokarhülse in seiner bestimmungsgemäßen Stellung (Einführstellung) festgelegt ist.

Zur Festlegung des Trokars in der bestimmungsgemäßen Stellung sind gemäß einer Weiterbildung der Erfindung an der Handhabe oder am Stab Mittel zum Festlegen des Trokars vorzugsweise am Haltearm vorgesehen. Da der Haltearm zur Führung und Festlegung von Instrumenten ohnehin vorgesehen ist, kann damit in vorteilhafter Weise auch der Trokar zumindest in Achsrichtung der Trokarhülse an dieser festgelegt werden. In Drehrichtung ergibt sich die Festlegung durch Formschluss aufgrund der polygonen Querschnittsform. Hierdurch kann auch sichergestellt werden, dass der oder die Wendel vom distalen Endabschnitt des Trokars stets bündig an die Wendel am Umfang des distalen Rohrabschnitts der Trokarhülse anschließen.

Um den Trokar mittels des Haltearms in der Trokarhülse festlegen zu können, ist vorteilhaft an der Handhabe, parallel zum Stab ein distalwärts gerichteter Haltestab vorgesehen, der nur etwa halb so lang wie der Stab zwischen Handhabe und dem distalen Endabschnitt des Trokars ist und der in den Haltearm einführbar und dort festlegbar ist.

Die Erfindung ist nachfolgend anhand eines in der Zeichnung dargestellten Ausführungsbeispiels näher erläutert. Es zeigen:
- Fig. 1: in stark vereinfachter schematischer Darstellung eine Trokarhülse gemäß der Erfindung mit eingesetzter Endoskopoptik in Seitenansicht,
- Fig. 2: einen Schnitt längs der Schnittlinie II-II in Fig. 1,
- Fig. 3: die Trokarhülse gemäß Fig. 1 im Längsschnitt mit darin eingesetztem Trokar,
- Fig. 4: eine Seitenansicht des Trokars,
- Fig. 5: einen Schnitt längs der Schnittlinie V-V in Fig. 4,
- Fig. 6: eine perspektivische Darstellung der in Fig. 1 dargestellten Dichtkappe von der Innenseite aus gesehen,
- Fig. 7: eine perspektivische Darstellung der Dichtkappe in einer weiteren Ausführungsform.
- Fig. 8: in stark vergrößerter Seitenansicht das distale Ende von Trokarhülse mit eingesetztem Trokar,
- Fig. 9: die Einzelheit IX in Fig. 8 in vergrößerter Darstellung.

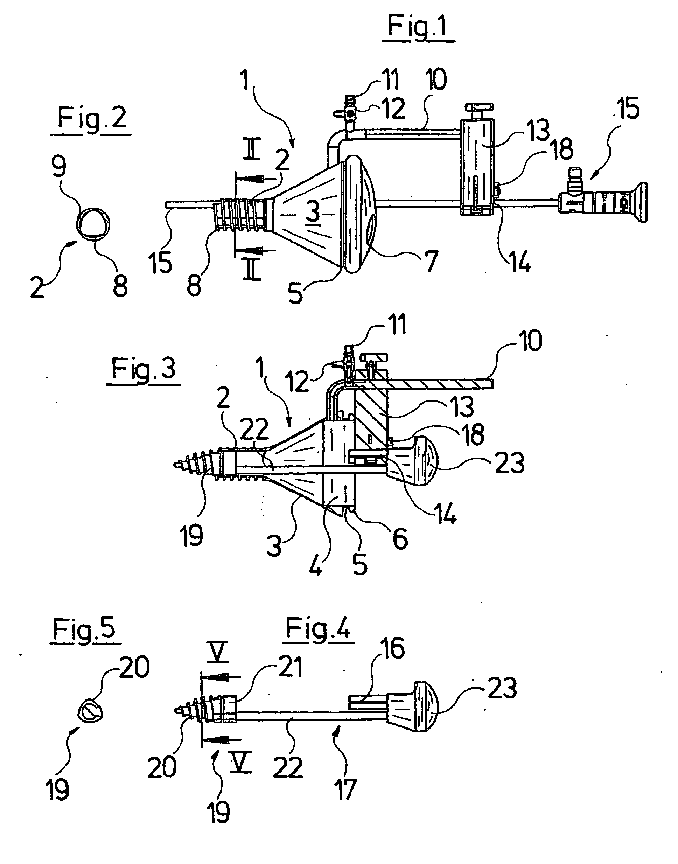

Die anhand der Figuren dargestellte Trokarhülse 1 weist einen distalen rohrförmigen Abschnitt 2 auf, an den proximalseitig (in Fig. 1 rechtsseitig) ein proximalwärts aufgeweiteter Abschnitt 3 anschließt, der wie die Darstellungen gemäß Fig. 1 und Fig. 3 verdeutlichen, etwa kegelstumpfförmig ausgebildet ist und bündig in den distalen Rohrabschnitt 2 übergeht, so dass sich die aus Fig. 2 ersichtliche trichterähnliche Form ergibt. An den proximalwärts aufgeweiteten Abschnitt 3 der Trokarhülse 1 schließt sich proximalseitig ein Endabschnitt 4 an, der an der Außenseite die Kegelstumpfform des Abschnitts 3 bis zu einer umlaufenden Nut 5 fortsetzt, an die endseitig ein Wulst 6 anschließt, der zusammen mit der Nut 5 zur lösbaren Befestigung einer Dichtkappe 7 vorgesehen ist. Die Innenseite des Endabschnitts 4 ist zylindrisch ausgebildet.

Der distale Rohrabschnitt 2 weist einen gerundeten dreieckigen Querschnitt auf, wie sich aus Fig. 2 ergibt. Dieser Rohrabschnitt 2 ist mit einer schraubenlinienförmig umlaufenden Wendel 8 versehen, die eine im Wesentlichen kreisrunde Außenkontur aufweist, wie Fig. 2 entnehmbar ist. Die Wendel 8 schließt an der Innenseite nahtlos an den Außenumfang des Rohrabschnitts 2 an, so dass sich im Bereich der Seiten 9 des dreieckigen Querschnitts eine größere Wendelbreite als in den Eckbereichen ergibt.

Im Bereich des Endabschnitts 4 schließt am Außenumfang radial ein Haltestab 10 an, der mit Abstand zum Außenumfang um 90° abgebogen und dort proximalwärts parallel zur Längsachse der Trokarhülse 1 verläuft. Der Haltestab 10 ist in dem Bereich, in dem er an den Endabschnitt 4 anschließt, innen hohl ausgebildet und über eine entsprechende Ausnehmung im Endabschnitt 4 mit dem Inneren der Trokarhülse 1 leitungsverbunden. An den hohlen Bereich des Haltestabs 10 ist unmittelbar hinter der 90°-Abbiegung eine Leitung 11 angeschlossen, in der ein Absperrventil 12 sitzt und die am freien Ende hinter dem Absperrventil 12 als Leitungsanschluss ausgebildet ist. Der Haltestab 10 ist im weiteren Verlauf aus Vollmaterial gebildet und hat einen sechseckigen Querschnitt. In diesem Bereich ist ein Haltearm 13 verschiebbar auf dem Haltestab 10 geführt, der an seinem freien Ende eine Aufnahme und Führungsöffnung 14 aufweist, in der wahlweise ein Instrument, z. B. die in Fig. 1 dargestellte Endoskopoptik 15 oder ein Haltestab 16 eines Trokars 17 festlegbar ist. Die Aufnahme und Führungsöffnung 14 verläuft parallel zur Längsachse der Trokarhülse und leicht versetzt zu dieser. Sie weist einen Exenterhebel 18 auf, mit dem das Instrument 15 bzw. der Haltestab 16 in dieser fixiert werden kann.

Der anhand der Fig. 4 und 5 dargestellte Trokar 17 weist einen distalen zu einer Spitze verjüngend zulaufenden Endabschnitt 19 auf, der von der Spitze zunächst kegelförmig aufgeweitet ausgebildet ist, wo er mit einer schraubenlinienförmig verlaufenden Wendel 20 versehen ist. Wie Fig. 5 verdeutlicht, weist der kegelförmige Teil einen runden Querschnitt auf, wohingegen die Wendel 20 so ausgebildet und angeordnet ist, dass sich in Achsrichtung des Trokars gesehen (Fig. 5) eine dreieckig gerundete Außenkontur ergibt entsprechend der Innenkontur des Rohrabschnitts 2 der Trokarhülse 1. Die Wendel 20 läuft proximalseitig in einen proximalen Teil 21 des Endabschnitts 19 aus, dessen Außenquerschnitt dem Innenquerschnitt des Rohrabschnitts 2 der Trokarhülse 1 entspricht und der in diesem Bereich mit einem O-Ring zur Abdichtung versehen sein kann. Wenn der Trokar 17 in seiner bestimmungsgemäßen Position in der Trokarhülse 1 sitzt (siehe Fig. 3), dann sitzt dieser proximale Teil 21 des Endabschnitts 19 innerhalb des Rohrabschnitts 2. Die Wendel 20 schließt dann bündig an die Wendel 8 an, die diese fortsetzt, wie aus Fig. 3 ersichtlich ist.

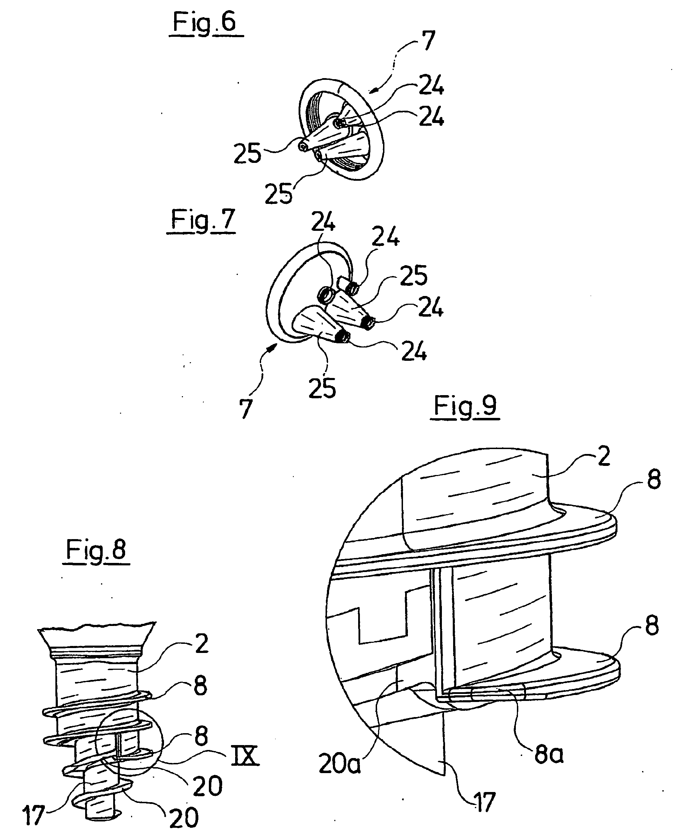

Statt eines bündigen Abschließens der aneinander anschließenden Wendelenden können diese auch wie anhand der Figuren 8 und 9 dargestellt um ein geringes Maß, beispielsweise entsprechend der Wendeldicke axial zueinander versetzt anschließen. Dabei schließt das distale Wendelende 8a der Trokarhülse 1 nach distalwärts ein kleines Stück versetzt zu dem proximalen Wendelende 20a der Wendel 20 des Trokars 17 an, wie dies aus Fig. 9 deutlich sichtbar ist.

In dieser Stellung (Fig. 3) gehalten wird der Endabschnitt 19 mittels eines proximal daran anschließenden Stabes 22, der bis zu einer Handhabe 23 am proximalen Ende des Trokars reicht. Parallel zu dem Stab 22 erstreckt sich von der Handhabe 23 distalwärts der Haltestab 16, der zur Befestigung in der Aufnahme und Führungsöffnung 14 des Haltearms 13 vorgesehen ist.

Wenn der Haltearm 13, der auf dem Haltestab 10 verschiebbar geführt ist, in der in Fig. 3 dargestellten Endstellung befindlich ist, in welcher der Haltearm 13 an der Leitung 11 anschlagartig anliegt und in dieser Stellung fixiert ist, dann ist die Aufnahme und Führungsöffnung 14 so angeordnet, dass bei Einsatz des Haltestabes 16 so weit bis die Handhabe 23 an der proximalen Seite des Haltearms 13 aufliegt, die Stellung erreicht ist, in welcher der Trokar 17 bestimmungsgemäß in der Trokarhülse 1 angeordnet ist, um seine Funktion als Trokar, also zum Durchdringen der Bauchdecke im Nabelbereich ausüben zu können.

Nachdem der Trokar 17 zusammen mit der Trokarhülse 1, wie es in Fig. 3 dargestellt ist, eingeführt und der distale Rohrabschnitt 2 mit seiner Wendel 8 in der Bauchdecke unter Komprimierung derselben festgelegt ist, kann der Trokar 17 durch einfaches Herausziehen in proximaler Richtung entfernt werden, wonach der Zugang zur Körperhöhle durch die Trokarhülse offengehalten wird.

Durch Anbringen der Dichtkappe 7, welche über den Wulst 6 gestülpt wird, so dass sie dichtend in die Nut 5 eingreift, wird die Trokarhülse 1 nach außen hin abgedichtet. Die elastische Dichtkappe 7 weist in dieser Ausführung insgesamt vier Öffnungen 24 auf, die zum Durchführen von Instrumenten vorgesehen sind. Dabei sind zwei dieser Öffnungen 24 mit selbsttätig schließenden Ventilen 25 versehen, die durch konische, in den trichterförmigen Bereich 3 der Trokarhülse 1 nach innen reichende Dichtungsabschnitte 25 gebildet sind, die sich bei bestehenden Pneumoperitoneum aufgrund des Überdrucks in der Trokarhülse 1 beim Entfernen des Instruments 15 selbsttätig verschließen und durch Einführen eines Instruments 15 wieder geöffnet werden können. Statt dieser selbsttätig schließenden elastischen durch die Form gebildeten Ventile 25 können auch Klappenventile oder mechanische Absperrvorrichtungen vorgesehen sein, die die entsprechende Öffnung nach Entnahme des Instruments verschließen. Über die Leitung 11 kann das Pneumoperitoneum unterstützt oder aufgebaut werden.

Die anhand der Figuren 6 und 7 dargestellten Dichtkappen 7 sind als Stülpkappen ausgebildet. Vorteilhaft können diese mittels eines Gelenks schwenkbar am Endabschnitt 4 der Trokarhülse 1 befestigt sein, so dass die Kappe verschwenkbar ist, was den Vorteil hat, dass die Anordnung der Öffnungen 24 in Bezug auf die Trokarhülse 1 festgelegt ist und daher nach dem Aufsetzen nicht mehr ausgerichtet werden muss.

### Bezugszeichenliste

- 1: Trokarhülse
- 2: distaler Rohrabschnitt
- 3: aufgeweiteter Abschnitt
- 4: Endabschnitt
- 5: Nut
- 6: Wulst
- 7: Dichtkappe
- 8: Wendel von 1
- 8a: distales Wendelende
- 9: Seiten des Dreieckquerschnitts 10 Haltestab
- 11: Leitung
- 12: Absperrventil
- 13: Haltearm
- 14: Aufnahme und Führungsöffnung
- 15: Endoskopoptik
- 16: Haltestab
- 17: Trokar
- 18: Exzenterhebel
- 19: distaler Endabschnitt von 17
- 20: Wendel von 17
- 20a: proximales Wendelende
- 21: proximaler Teil von 19
- 22: Stab
- 23: Handhabe
- 24: Öffnungen
- 25: Ventile

## Patentansprüche

1. Trokarhülse mit einem distalen rohrförmigen Abschnitt (2) und einem proximalseitig daran anschließenden und proximalwärts aufgeweiteten Abschnitt (3), mit mindestens einer am Umfang des distalen Rohrabschnitts (2) schraubenlinienförmig angeordneten Wendel (8) mit kreisrunder Außenkontur, **dadurch gekennzeichnet, dass** der distale Rohrabschnitt (2) einen gerundeten polygonförmigen Querschnitt aufweist.

2. Trokarhülse nach Anspruch 1, **dadurch gekennzeichnet, dass** die Wendel (8) über ihre gesamte Länge abstandsfrei an den Außenumfang des distalen Rohrabschnitts (2) anschließt.

3. Trokarhülse nach Anspruch 1 oder 2, **dadurch gekennzeichnet dass** der distale Rohrabschnitt (2) einen gerundeten dreieckigen Querschnitt aufweist.

4. Trokarhülse nach einem der vorhergehenden Ansprüche, **dadurch gekennzeichnet, dass** proximalseitig Mittel (5, 6) zum lösbaren Anbringen von Dichtmitteln (7), vorzugsweise einer Dichtkappe (7) vorgesehen sind, welche die Trokarhülse (1) proximalseitig nach außen hin abdichtet und mindestens eine selbsttätig schließende oder verschließbare Öffnung (24) zum Durchführen eines Instrumentes (15) aufweist.

5. Trokarhülse nach einem der vorhergehenden Ansprüche, **dadurch gekennzeichnet, dass** sich an den aufgeweiteten Abschnitt (3) proximalseitig ein Endschnitt (19) anschließt, welcher mit einem umlaufenden Wulst (6) versehen ist, der von der Dichtkappe (7) übergriffen ist.

6. Trokarhülse nach einem der vorhergehenden Ansprüche, **dadurch gekennzeichnet, dass** ein Haltestab (10) vorgesehen ist, der vorzugsweise an der Außenseite des Endabschnitts (19) anschließt, abgebogen ist und parallel zur Längsachse der Trokarhülse (1) proximalwärts geführt ist, mit einem verstellbar darauf angeordneten Haltearm (13), der zur Führung und Fixierung eines durch die Trokarhülse (1) geführten Instrumentes (15) angeordnet und ausgebildet ist.

7. Trokarhülse nach einem der vorhergehenden Ansprüche, **dadurch gekennzeichnet, dass** der Haltestab (10) zumindest abschnittsweise hohl ausgebildet ist, eine Leitungsverbindung ins Innere der Trokarhülse (1) bildet und einen vorzugsweise absperrbaren Leitungsanschluss (11) aufweist.

8. Trokarhülse nach einem der vorhergehenden Ansprüche, **dadurch gekennzeichnet, dass** ein Trokar (17) vorgesehen ist, der in der Trokarhülse (1) festlegbar ist und welcher einen distalen zu einer Spitze hin verjüngten Enabschnitt (19) aufweist, dessen Außenkontur dem Innenquerschnitt des distalen Rohrabschnitts (2) entspricht und der mit mindestens einer Wendel (20) versehen ist, die bündig an die Wendel (8) am Umfang des distalen Rohrabschnitts (2) anschließt wenn der Trokar (17) in der Trokarhülse (1) in seiner bestimmungsgemäßen Stellung festgelegt ist.

9. Trokarhülse nach einem der vorhergehenden Ansprüche, **dadurch gekennzeichnet, dass** ein Trokar (17) vorgesehen ist, der in der Trokarhülse (1) festlegbar ist und welcher einen distalen zu einer Spitze hin verjüngten Endabschnitt (19) aufweist, dessen Außenkontur dem Innenquerschnitt des distalen Rohrabschnitts (2) entspricht und der mit mindestens einer Wendel (20) versehen ist, die an die Wendel (8) am Umfang des distalen Rohrabschnitts (2) anschließt wenn der Trokar (17) in der Trokarhülse (1) in seiner bestimmungsgemäßen Stellung festgelegt ist, wobei das distale Ende (8a) der Wendel (8a) am Umfang des distalen Rohrabschnitts (2) nach distalwärts versetzt gegenüber dem proximalen Ende(20a) der Wendel (20) des Trokars (17) angeordnet ist.

10. Trokarhülse nach einem der vorhergehenden Ansprüche, **dadurch gekennzeichnet, dass** der Trokar (17) einen proximalseitig an den verjüngten Endabschnitt (19) anschließenden Stab (22) aufweist mit endseitiger Handhabe (23) aufweist, der so dimensioniert ist, dass die Handhabe (23) die Trokarhülse (1) proximalseitig überragt wenn der Trokar (17) in der Trokarhülse (1) in seiner bestimmungsgemäßen Stellung festgelegt ist.

11. Trokarhülse nach einem der vorhergehenden Ansprüche, **dadurch gekennzeichnet, dass** an der Handhabe (23) oder dem Stab (22) Mittel zum Festlegen des Trokars (17) an der Trokarhülse (1) vorgesehen sind, vorzugsweise an der Handhabe (23) ein distalwärts gerichteter Haltestab (16) vorgesehen ist, der im Haltearm (13) festlegbar ist.

## Claims

1. A trocar sleeve with a distal, tubular section (2) and with a section (3) which connects thereto at the proximal side and which is widened proximally, with at least one spiral (8) with a circular contour, which is arranged helically on the periphery of the distal tube section (2), **characterised in that** the distal tube section (2) has a rounded, polygonal cross section.

2. A trocar sleeve according to claim 1, **characterised in that** the spiral (8) over its entire length connects to the outer periphery of the distal tube section (2) in a distance-free manner.

3. A trocar sleeve according to claim 1 or 2, **characterised in that** the distal tube section (2) comprises a rounded triangular cross section.

4. A trocar sleeve according to one of the preceding claims, **characterised in that** means (5, 6) for the releasable attachment of sealing means (7), preferably of a sealing cap (7), are provided on the proximal side, said sealing means sealing the trocar sleeve (1) on the proximal side to the outside and comprising at least one automatically closing or closable opening (24) for leading through an instrument (15).

5. A trocar sleeve according to one of the preceding claims, **characterised in that** an end-section (4) connects onto the proximal side to the widened section (3) and is provided with a peripheral bead (6), over which the sealing cap (7) engages.

6. A trocar sleeve according to one of the preceding claims, **characterised in that** a holding rod (10) is provided, which preferably connects on the outer side of the end-section (4), is bent and is guided proximally and parallel to the longitudinal axis of the trocar sleeve (1), with a holding arm (3) which is arranged thereon in an adjustable manner and which is arranged and designed for the guiding and fixation of an instrument (15) led through the trocar sleeve (1).

7. A trocar sleeve according to one of the preceding claims, **characterised in that** the holding rod (10) at least in sections is designed in a hollow manner, forms a conduit connection into the inside of the trocar sleeve (1) and comprises a conduit connection (11) which can preferably be shut off.

8. A trocar sleeve according to one of the preceding claims, **characterised in that** a trocar (1 7) is provided, which can be fastened in the trocar sleeve (1) and which comprises a distal end-section (19) which is tapered into a tip and whose outer contour corresponds to the inner cross section of the distal tube section (2) and which is provided with at least one spiral (20) which is connecting to the spiral (8) on the periphery of the distal tube section (2) in an aligned manner, when the trocar (17) is fixed in the trocar sleeve (1) in its intended position.

9. A trocar sleeve according to one of the preceding claims, **characterised in that** a trocar (17) is provided, which can be fastened in the trocar sleeve (1) and which comprises a distal end-section (19) which is tapered into a tip and whose outer contour corresponds to the inner cross section of the distal tube section (2) and which is provided with at least one spiral (20) connecting to the spiral (8) on the periphery of the distal tube section (2), when the trocar (17) is fastened in the trocar sleeve (1) in its intended position, wherein the distal end (8a) of the spiral (8) on the periphery of the distal tube section (2) is arranged distally displaced with respect to the proximal end (20a) of the spiral (20) of the trocar (17).

10. A trocar sleeve according to one of the preceding claims, **characterised in that** the trocar (1 7) comprises a rod (22) which connects on the proximal side to the tapered end-section (19), said rod having an end-side handle (23) which is dimensioned such that the handle (23) projects beyond the trocar sleeve (1) on the proximal side, when the trocar (17) is fixed in the trocar sleeve (1) in its intended position

11. A trocar sleeve according to one of the preceding claims, **characterised in that** means for fixing the trocar (17) on the trocar sleeve (1) are provided on the handle (23) or on the rod (22), and a distally directed holding rod (16) is provided, preferably on the handle (23) and can be fastened in the holding arm (13).

## Revendications

1. Chemise de trocart munie d'un segment tubulaire distal (2) et d'un segment côté proximal (3), raccordé au premier et élargi dans la direction proximale, et d'au moins une spire (8) à contour extérieur circulaire, disposée en hélice à la périphérie du segment tubulaire distal (2), **caractérisée en ce que** le segment tubulaire distal (2) présente une section transversale polygonale arrondie.

2. Chemise de trocart selon la revendication 1, **caractérisée en ce que** la spire (8) se raccorde directement à la périphérie extérieure du segment tubulaire distal (2) sur toute sa longueur.

3. Chemise de trocart selon la revendication 1 ou 2, **caractérisée en ce que** le segment tubulaire distal (2) présente une section transversale en forme de triangle arrondi.

4. Chemise de trocart selon l'une des revendications précédentes, **caractérisée en ce que**, sur le côté proximal sont prévus des moyens (5, 6) servant à monter de façon démontable des moyens d'étanchéité (7), de préférence un capuchon de fermeture étanche (7) qui ferme la chemise de trocart (1) de façon étanche par rapport à l'extérieur sur le côté proximal et présente au moins une ouverture (24) à fermeture automatique ou pouvant être obturée, prévue pour l'introduction d'un instrument (15).

5. Chemise de trocart selon l'une des revendications précédentes, **caractérisée en ce qu'**au segment élargi (3) se raccorde sur le côté proximal un segment terminal (4) muni d'un bourrelet périphérique (6) qui est emboîté par le capuchon de fermeture étanche (7).

6. Chemise de trocart selon l'une des revendications précédentes, **caractérisée en ce qu'**il est prévu une barre de maintien (10) qui se raccorde de préférence au côté extérieur du segment terminal (4), est coudée et s'étend parallèlement à l'axe longitudinal de la chemise de trocart (1) dans la direction proximale, et sur laquelle un bras de maintien (13), monté de façon réglable, est disposé et configuré pour guider et bloquer un instrument (15) introduit à travers la chemise de trocart (1).

7. Chemise de trocart selon l'une des revendications précédentes, **caractérisée en ce que** la barre de maintien (10) est creuse, au moins par sections, forme un raccordement de conduite donnant sur le volume intérieur de la chemise de trocart (1), et présente un raccord de conduite (11) de préférence obturable.

8. Chemise de trocart selon l'une des revendications précédentes, **caractérisée en ce qu'**il est prévu un trocart (17) qui peut être fixé dans la chemise de trocart (1) et qui présente un segment terminal distal (19) qui se rétrécit en une pointe, dont le contour extérieur correspond à la section intérieure du segment tubulaire distal (2) et qui est muni d'au moins une spire (20) qui se raccorde précisément à la spire (8) présente sur la périphérie du segment tubulaire distal (2) lorsque le trocart (17) est fixé dans la chemise de trocart (1), dans sa position fonctionnelle.

9. Chemise de trocart selon l'une des revendications précédentes, **caractérisée en ce qu'**il est prévu un trocart (17) qui peut être fixé dans la chemise de trocart (1) et qui présente un segment terminal distal (19) se rétrécissant en une pointe, dont le contour extérieur correspond à la section intérieure du segment tubulaire distal (2) et qui est muni d'au moins une spire (20) qui se raccorde à la spire (8) présente sur la périphérie du segment tubulaire distal (2) lorsque le trocart (17) est fixé dans la chemise de trocart (1), dans sa position fonctionnelle, l'extrémité distale (8a) de la spire (8a) présente à la périphérie du segment tubulaire distal (2) étant décalée dans la direction distale par rapport à l'extrémité proximale (20a) de la spire (20) du trocart (17).

10. Chemise de trocart selon l'une des revendications précédentes, **caractérisée en ce que** le trocart (1 7) présente une barre (22) qui se raccorde au segment terminal rétréci (19) sur le côté proximal et qui est munie d'une poignée terminale (23) dimensionnée de telle manière que la poignée (23) déborde au-delà de la chemise de trocart (1) sur le côté proximal lorsque le trocart (17) est fixé dans la chemise de trocart (1), dans sa position fonctionnelle.

11. Chemise de trocart selon l'une des revendications précédentes, **caractérisée en ce que** sont prévus sur la poignée (23) ou sur la barre (22) des moyens servant à fixer le trocart (17) à la chemise de trocart (1), **en ce qu'**est prévue de préférence au niveau de la poignée (23) une barre de maintien (16) dirigée dans la direction distale et qui peut être bloquée dans le bras de maintien (13).
